# EUROPEAN PATENT APPLICATION

(11) **EP 0 712 640 A1**
(43) Date of publication of application: **22.05.1996**
(21) Application number: 95307611.4
(22) Date of filing: 26.10.1995
(51) Int. Cl.: A61M 39/24, F16K 15/14

(54) **Medical apparatus with valved fluid line**

(30) Priority: 19.11.1994 GB 9423386
(71) Applicant: Smiths Industries Public Limited Company, London, NW11 8DS (GB)
(72) Inventor: Carter, Roland H.C., Hythe, Kent, CT2 6DW (GB)
(74) Representative: Flint, Jonathan McNeill

(57) **Abstract**

Medical apparatus has a liquid reservoir 3 connected to a pump 4 that pumps liquid out of the reservoir. A normally-closed valve 33, such as included in a drip chamber 30, is connected at the outlet of the reservoir so that the valve prevents flow of liquid out of the reservoir except when the pump 4 is in operation. The valve 33 has a resilient, mushroom shape element 34 extending through a fluid passage 36 in a flange 37 in the valve. One end 38 of the valve element 34 contacts a surface of the flange to prevent fluid passage unless it is deflected by pressure of fluid from the pump 4.

## Description

This invention relates to medical apparatus of the kind including a liquid reservoir and a pump for pumping liquid out of the reservoir.

Various forms of medical apparatus include a fluid supply, some of which include a pump controlling the rate of fluid supply. Examples of such apparatus include humidifiers in which water is supplied via a pump at a controlled rate to an evaporator or nebulizer. The pump is connected between the humidifier and a water reservoir, such as a suspended bag of sterile water, so that the water flows by a combination of gravity and motive power delivered by the pump. Flow rate is controlled by altering the speed of the pump, which may be a peristallic pump.

One problem with this is that, if the pump should fail, or if the tubing between the bag and the humidifier should became detached from the pump, there can be a continuous, uncontrolled flow of water. This can be dangerous because the water may flow into the respiratory passages of the patient.

It is an object of the present invention to provide medical apparatus that can be used to alleviate this problem.

According to the present invention there is provided medical apparatus of the above-specified kind, characterised in that the apparatus includes a normally-closed valve that prevents flow out of the reservoir except when pumped out of the reservoir by the pump.

The valve may be connected between the pump and the reservoir, such as at the outlet of the reservoir. The valve may be mounted in a drip chamber of the apparatus. The valve preferably includes a flange extending across the inside of the valve, a fluid passage through the flange, and a valve member retained on the flange. The valve member preferably extends through the fluid passage and may be of a resilient material having enlarged portions at opposite ends that to retain the valve member on the flange. The enlarged portion at one end of the valve member may engage and seal with a surface of the flange and be deflected away from the surface by pressure of fluid. The enlarged portion at one end preferably includes a peripheral wall that engages the flange, the fluid passage opening within the wall. The pump may be a peristaltic pump.

A medical humidifier system including apparatus according to the present invention, will now be described, by way of example, with reference to the accompanying drawings, in which:
- Figure 1: illustrates the system schematically; and
- Figure 2: is a cross-section elevation of a part of the system in greater detail.

The system includes a humidifier unit 1 connected into a respiratory circuit 2 and supplied with water from a fluid reservoir in the form of a flexible bag 3, via a pump unit 4 and tubing 5.

The humidifier unit 1 may be of a bi-directional kind in which both inhaled and exhaled respiratory gases flow through the unit, in opposite directions. The unit 1 includes an outer transparent housing 10 with a coupling 11 at one end connected via tubing 12 to a ventilator machine 13. A coupling 14 at the opposite end of the housing 10 is connected to a face mask 15, tracheal tube or the like, via tubing 16. The housing 10 includes a conventional heat and moisture exchange (HME) element 17, such as of a hygroscopic paper, and an absorbent wick 18 located between the HME element and the patient coupling 14. The wick 18 surrounds an electrical heating element 19 that is supplied with power from a heater control circuit 40 in the pump unit 4. The humidifier unit 1 has a water inlet conduit 20 projecting from the housing 10 in the region of the wick 18. The conduit 20 is connected to one end of the tubing 5 so that water flowing into the conduit is absorbed by the wick 18 and evaporated by the heating element 18 to humidify gases supplied to the patient. The humidifier unit 1 is conventional and as described in WO 91/19527. Alternative humidifiers can also be used.

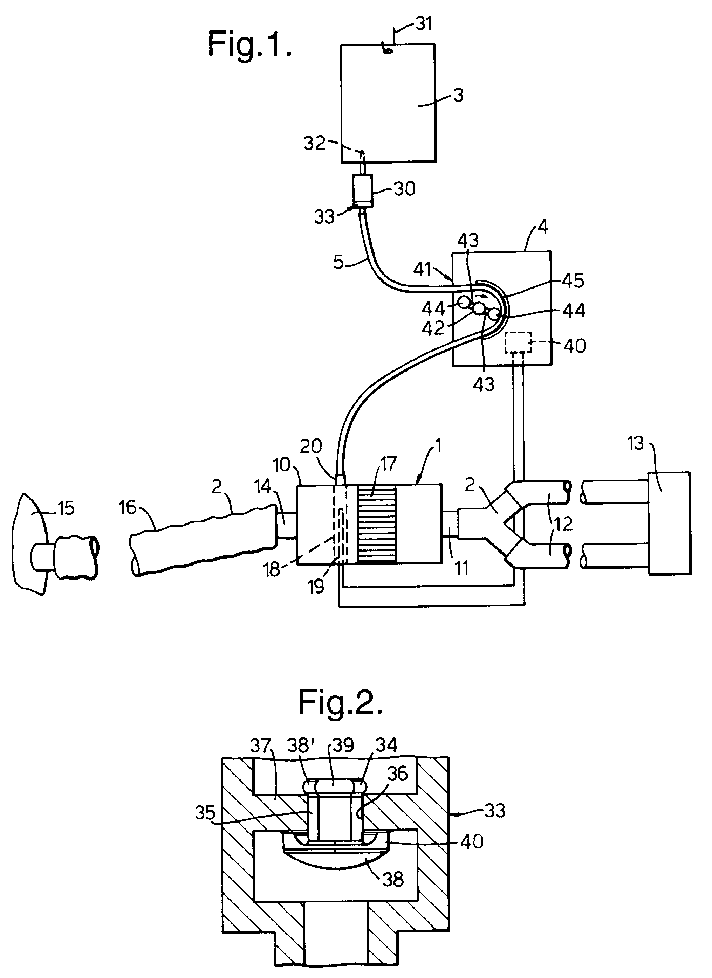

The water supply tubing 5 is of a resilient plastics material, such as PVC or silicone, which will operate with a conventional peristaltic pump. The tubing 5 extends in one continuous length to a drip chamber 30 connected at an outlet of the bag 3. The bag 3 is of a conventional kind typically containing about 250ml or 1l of sterile water and suspended by a hook 31. Connection to the bag is made by a spike 32 at the upper end of the drip chamber 30. The lower end of the drip chamber 30 contains a normally-closed valve 33. The valve 33 has a resilient, rubber valve member 34 in the shape of an inverted mushroom. The stem 35 of the valve member 34 extends through a fluid passage or aperture 36 in a flange 37 and is retained on the flange by a lower, enlarged sealing head 38 and an upper retaining head 38'. The lower, downstream head 38 has a peripheral wall 40 of cylindrical shape, which is shown in Figure 2 cut away around a part of its circumference to reveal the stem although, in fact, the wall forms a complete circle. Two flats 39 extend along the stem 35 and the upper head 38' so that fluid can flow through the aperture around the stem. In its natural state, the upper edge of the wall 40 of the lower head 38 seals with the underside of the flange 37, the length of the stem 35 being such that it is stretched by the thickness of the flange to hold the two heads 38 and 38' firmly against the flange. In this state, the valve 33 prevents flow of water from the drip chamber 30 and bag 3. The valve 33 is opened when negative pressure is applied by the pump unit 4 to the lower side of the valve, since this will deflect the wall 40 of the lower head 38 away from contact with the flange 37. The fluid passage through the flange need not be provided by the same aperture 36 by which the valve member 34 is retained, instead, alternative fluid passages could be formed opening on the lower surface of the flange within the wall 40.

The pump unit 4 contains a conventional peristaltic pump 41 having rotating shaft 42 with two or more radial arms 43 each of which has a wiping finger 44 at its outer end. A semi-circular channel 45 is located coaxially of the shaft 42 to retain a part of the length of the water supply tubing 5. As the shaft 42 rotates clockwise, the fingers 44 squeeze the tubing 5 against the channel 45 and force the water in the tubing in the direction of rotation, towards the humidifier unit 1. The rate of flow of water through the tubing 5 is controlled by the speed of rotation of the pump. When the pump is stationary, no water can flow along the tubing 5 because it is squeezed closed between the channel 45 and one of the fingers 44.

The tubing 5 can be easily removed from the pump 4 by pulling away from between the channel 45 and the fingers 44. In this way, the tubing can be replaced before it loses its resilience and the performance of the pump is degraded.

In setting up the humidifier system, the user must thread the tubing 5 into the pump 41. There is a risk, therefore, that this will be overlooked and that the bag 3 will be connected directly to the humidifier unit 1. If the valve 33 were not present, water would flow directly from the bag 3 to the humidifier 1. Although the drip chamber 30 would reduce this flow, it could still be too great in some situations and lead to over saturation of the wick 18. Water could then collect in the housing 10 and might flow into the ventilation circuit 2. The valve 33 of the present invention, however, prevents any flow of water into the tubing 5 unless a negative pressure is exerted on the tubing by the pump 4.

It will be appreciated that different forms of pump and valve could be used and that the invention could be applied to other medical apparatus including fluid supply means, such as infusion pumps. The valve could be located at other points in the apparatus, such as, close to the humidifier unit 1. In other apparatus according to the invention, the pump could be connected at an inlet of the liquid reservoir to pump out liquid in the reservoir by pumping in gas. Alternatively, the pump could comprise a device that applies pressure to the walls of a flexible liquid reservoir. The pump could be a syringe-like device with the liquid reservoir being formed between the barrel and the plunger.

## Claims

1. Medical apparatus including a liquid reservoir (3) and a pump (4) for pumping liquid out of the reservoir, characterised in that the apparatus includes a normally-closed valve (33) that prevents flow of liquid out of the reservoir (3) except when pumped out of the reservoir by the pump (4).

2. Medical apparatus according to Claim 1, characterised in that the valve (33) is connected between the pump (4) and the reservoir (3).

3. Medical apparatus according to Claim 1 or 2, characterised in that the valve (33) is connected at an outlet of the liquid reservoir (3).

4. Medical apparatus according to any one of the preceding claims including a drip chamber (30), and characterised in that the valve (33) is mounted in the drip chamber (33).

5. Medical apparatus according to any one of the preceding claims, characterised in that the valve (33) includes a flange (37) extending across the inside of the valve, a fluid passage (36) through the flange, and a valve member (34) retained on the flange (37).

6. Medical apparatus according to Claim 5, characterised in that the valve member (34) extends through the fluid passage (36).

7. Medical apparatus according to Claim 5 or 6, characterised in that the valve member (34) is of a resilient material and has enlarged portions (38, 38') at opposite ends that retain the valve member (34) on the flange (37).

8. Medical apparatus according to Claim 7, characterised in that the enlarged portion (38) at one end of the valve member (34) engages and seals with a surface of the flange (37) and is deflected away from the flange surface by pressure of fluid.

9. Medical apparatus according to Claim 8, characterised in that the enlarged portion (38) at one end includes a peripheral wall (40) that engages the flange (37), and that the fluid passage (36) opens within the wall (40).

10. Medical apparatus according to any one of the preceding claims, characterised in that the pump is a peristaltic pump (4).
